# EUROPÄISCHE PATENTSCHRIFT

(11) **EP 4 004 524 B1**
(45) Veröffentlichungstag und Bekanntmachung des Hinweises auf die Patenterteilung: **19.07.2023**
(21) Anmeldenummer: 20740629.9
(22) Anmeldetag: 20.07.2020
(51) Int. Cl.: G01N 21/33, G01N 21/3504, G01N 21/41, G01N 21/47, G01N 21/94, G01N 33/00

(54) **VERFAHREN UND VORRICHTUNG ZUR QUALITÄTSÜBERWACHUNG UND FESTSTELLUNG EINER KONTAMINATION EINES RAUMS**
METHOD AND DEVICE FOR QUALITY MONITORING AND FOR DETERMINING THE CONTAMINATION OF A SPACE
PROCÉDÉ ET DISPOSITIF DE SURVEILLANCE DE LA QUALITÉ ET DE DÉTERMINATION D'UNE CONTAMINATION D'UN ESPACE

(30) Priorität: 26.07.2019 EP 19188660
(43) Veröffentlichungstag der Anmeldung: 01.06.2022
(73) Patentinhaber: Currenta GmbH & Co. OHG, 51373 Leverkusen (DE)
(72) Erfinder: ALTHOFF, Marc André, 51067 Köln (DE); VON STUMBERG, Axel, 51467 Bergisch Gladbach (DE)
(74) Vertreter: Michalski Hüttermann & Partner Patentanwälte mbB
(86) Internationale Anmeldenummer: PCT/EP2020/070408
(87) Internationale Veröffentlichungsnummer: WO 2021/018647

(56) Entgegenhaltungen:
- US-A1- 2009 018 780
- US-B1- 7 383 129

## Beschreibung

Die vorliegende Erfindung betrifft ein Verfahren zur Qualitätsüberwachung und Feststellung einer Kontamination eines Raums, sowie eine Vorrichtung zur Qualitätsüberwachung und Feststellung einer Kontamination eines Raums.

Ein Fluid in einem Raum, beispielsweise die Luft in Lufträumen, beispielsweise über Städten oder Industrieanlagen, kann abhängig von einer Vielzahl an Faktoren unterschiedliche Qualitäten aufweisen. Beeinträchtigungen der Luftqualität können beispielsweise durch lokale Emissionen einer Vielzahl verschiedener Substanzen auftreten oder aber durch Wind herangetragen werden. Solche Beeinträchtigungen können für den Menschen über den Geruch wahrnehmbar oder auch optisch erkennbar sein. Der Mensch kann derartige Vorkommnisse aber zumeist erst erkennen, wenn die Luftqualität schon stark beeinträchtigt ist. Einige Beeinträchtigungen kann der Mensch dabei überhaupt nicht selbst erkennen, andere Beeinträchtigungen sind zwar sehr deutlich wahrnehmbar aber nicht gefährlich. Zudem können derartige Beeinträchtigungen lokal sehr eng begrenzt sein, so dass die Beeinträchtigung nur an wenigen Orten beobachtbar ist.

Zum Schutz der Bevölkerung ist es deshalb in einigen Gebieten ein Bedürfnis die Qualität eines Raums kontinuierlich und raumdeckend zu überwachen.

Systeme zur Qualitätsüberwachung sind an sich bekannt.

Beispielsweise beschreibt die US 2019/0113445 A1 ein System zur Luftverschmutzungsüberwachung und ein Verfahren zur Luftverschmutzungsüberwachung, wobei eine Vielzahl von Überwachungsvorrichtungen in unterschiedlichen Höhen und Stufen angeordnet ist und Infrarotreflektierende Anordnungen der Überwachungsvorrichtung in unterschiedlichen Höhen angeordnet sind, um Infrarot-Spektraldaten von chemischen Bestandteilen der Luftmasse zu sammeln, die in unterschiedlich hohen Lufträumen diffundieren. Die in der Luftmasse enthaltenen Verschmutzungsfaktoren und der Verschmutzungsgrad jedes Faktors werden mithilfe des IR-Spektrums für die qualitative und quantitative Analyse des Verschmutzungsfaktors ermittelt, wodurch eine kontinuierliche Überwachung in einem bestimmten Bereich ermöglicht wird.

Um die Qualität mit einer hohen Sensibilität und Genauigkeit zu überwachen wird zumeist eine große Zahl an Sensoren eingesetzt. Diese sind jedoch zum Teil sehr teuer und werden deswegen nicht raumdeckend oder zumindest nicht in einer ausreichenden Dichte angebracht. Es kann aufwändig sein, die verschiedenen Sensoren an den unterschiedlichen Positionen anzubringen oder überhaupt geeignete Positionen auszumachen. Zudem kann ein Nachteil sein, dass die Sensoren trotz sorgfältiger Positionierung für manche Orte und Substanzen im Raum blind sind oder eben zu grobmaschig verteilt sind. Zur besonders genauen Bestimmung der Qualität werden zudem Sensoren benötigt, die nur lokal messen können. Bekannte Verfahren und Vorrichtungen zur Qualitätsüberwachung von Räumen bieten deswegen noch Verbesserungspotential.

US 7 383 129 B1 beschreibt ein System und ein Verfahren zur Visualisierung einer Szene, die auf Substanzkontamination überwacht wird. Die Szene wird mit einem Stoffdetektor gescannt, um einen Stoff wie eine schädliche chemische oder biologische Substanz zu erkennen. Die Position des Stoffes wird bestimmt. Es werden grafische Elementdaten generiert, die die erkannte Substanz in der Szene darstellen. Eine angezeigte Ansicht der Szene wird mit den grafischen Elementdaten basierend auf der Position der detektierten Substanz ergänzt, um die detektierte Substanz in der Ansicht der Szene anzuzeigen und dem Benutzer den Ort und den Typ der detektierten Substanz in Bezug auf die Umgebung anzuzeigen.

US 2009/018780 A1 beschreibt ein Verfahren und ein System zum Erhalten von Informationen über Verunreinigungen in der Umgebungsluft. Mehrere Detektionssysteme messen die Umgebungsluft in Echtzeit auf Verunreinigungen. Jedes der mehreren Nachweissysteme analysiert Schadstoffe auf gefährliche Substanzen. Die Mehrfacherkennungssysteme übertragen Informationen über die gefährlichen Substanzen an ein Satellitenüberwachungssystem. Das Satellitenüberwachungssystem empfängt die übertragenen Informationen. Das Satellitenüberwachungssystem verpackt die von jedem der Erfassungssysteme übertragenen Informationen und analysiert die verpackten Informationen. Das Satellitenüberwachungssystem überträgt die analysierten Informationen an eine Konsole, die auf einem Computergerät an einer Kommandostation installiert ist. Die Konsole empfängt die übertragenen Informationen vom Satellitenüberwachungssystem und kommuniziert Benutzerinteraktion basierend auf den übertragenen Informationen an das Satellitenüberwachungssystem.

Die vorliegende Erfindung hat sich die Aufgabe gestellt, ein verbessertes Verfahren und eine verbesserte Vorrichtung zur Qualitätsüberwachung und Feststellung einer Kontamination eines Raums bereitzustellen.

Erfindungsgemäß gelöst wird diese Aufgabe durch das Verfahren nach Anspruch 1 und das System nach Anspruch 12. Mit der Erfindung wird ferner die Verwendung des Verfahrens nach Anspruch 15 beansprucht.

Vorteilhafte Weiterbildungen sind in den Unteransprüchen angegeben.

Mit der Erfindung wird ein Verfahren zur Qualitätsüberwachung und Feststellung einer Kontamination eines Raums vorgeschlagen, wobei der Raum ein durch Raumkoordinaten bestimmbares Volumen ist, der ein Fluid aufweist oder der im Wesentlichen mit einem Fluid gefüllt ist.

Das Verfahren weist die Verfahrensschritte auf,
a) Messen einer Mehrzahl von Qualitätsparameter-Istwerten in einem Raum mit einem Sensorsystem, wobei der Raum ein durch Raumkoordinaten bestimmbares Volumen ist, welcher ein Fluid aufweist oder der im Wesentlichen mit einem Fluid gefüllt ist, wobei der Istwert von zumindest einem Qualitätsparameter an einer Mehrzahl von Messorten gemessen wird, wobei das Sensorsystem eine Mehrzahl von Sensoren umfasst, wobei die Sensoren mobile Sensoren sind, oder zumindest ein Sensor ein stationärer Sensor und zumindest ein Sensor ein mobiler Sensor ist, wobei jeder Sensor an einer mittels Raumkoordinaten bestimmten Position des Messortes den zumindest einen Qualitätsparameter-Istwert misst,
b) Übertragen der an einer Mehrzahl von Messorten gemessenen Qualitätsparameter-Istwerte an ein Datenverarbeitungssystem,
c) Abgleich der an den jeweiligen Messorten gemessenen Qualitätsparameter-Istwerte mit in dem Datenverarbeitungssystem für die jeweiligen Messorte hinterlegten Sollwerten,
d) Bewegen des zumindest einen mobilen Sensors mit einem Steuersystem an einen von dem vorherigen Messort des zumindest einen mobilen Sensors beabstandeten Messort,
wobei der zumindest eine mobile Sensor verschiedene Messorte nach einem in dem Datenverarbeitungssystem hinterlegten Bewegungsplan oder nach einem neu berechneten Bewegungsplan ansteuert, wobei bei einem Störungsfall, bei dem wenigstens ein Istwert von dem für den jeweiligen Messort definierten Sollwert abweicht, zumindest ein mobiler Sensor mit dem Steuersystem von dem hinterlegten Bewegungsplan abweichend bewegt wird und in dem Raum um die Position des Messortes mit der detektierten Istwertabweichung herum, zusätzliche Qualitätsparameter-Istwerte an zusätzlichen Messorten misst und an das Datenverarbeitungssystem überträgt, wobei das Datenverarbeitungssystem die räumliche Ausbreitung, Art der Kontamination und/oder Konzentration der jeweiligen Kontamination des Raums bestimmt.

Unter einem Raum ist im Sinne der vorliegenden Erfindung ein durch Raumkoordinaten bestimmbares Volumen zu verstehen. Der Raum kann teilweise oder vollständig umschlossen sein, beispielsweise von einem Boden, von Wänden und/oder von einer Decke, kann aber auch keine Abgrenzung aufweisen. Unter einem Raum ist dabei zu verstehen, dass der Raum zumindest ein Fluid aufweist oder der Raum im Wesentlichen mit einem Fluid gefüllt ist.

Gemäß einer Ausführungsform kann der Raum zumindest ein Fluid aufweisen. Gemäß einer weiteren Ausführungsform kann der Raum zumindest im Wesentlichen mit einem Fluid gefüllt sein.

Im Wesentlichen im Sinne dieser Erfindung bedeutet, dass das Fluid den größten Volumenanteil des Raumes einnimmt oder mehr als 50 Volumen % des Raumes ein Fluid aufweist.

Ein Fluid im Sinne dieser Erfindung ist ein Gas, eine Flüssigkeit oder ein Gemisch umfassend ein Gas und eine Flüssigkeit. Vorzugsweise kann ein Fluid Wasser, Wasserdampf und/oder Luft aufweisen. Erfindungsgemäß kann ein Fluid beispielsweise im Wesentlichen Wasser oder Luft aufweisen. Bevorzugt kann dabei vorgesehen sein, dass der Raum ein Luftraum und/oder der Raum einen Wasserbereich aufweist, vorzugsweise ein Luftraum, und der Qualitätsparameter ein Luftqualitätsparameter und/oder Wasserqualitätsparameter, vorzugsweise ein Luftqualitätsparameter, ist. Dabei kann insbesondere vorgesehen sein, dass das Fluid des Raumes mit dem Fluid außerhalb des Raumes im Stoffaustausch steht.

Unter einer Anzahl ist im Sinne der vorliegenden Erfindung eine Zahl von zumindest eins zu verstehen. Unter einer Mehrzahl ist im Sinne der vorliegenden Erfindung eine Zahl von zumindest zwei zu verstehen.

Unter einem Qualitätsparameter ist im Sinne der vorliegenden Erfindung ein Parameter zu verstehen, der direkt oder indirekt auf die Qualität des im Raum befindlichen Mediums schließen lässt. Es kann besonders bevorzugt vorgesehen sein, dass der Qualitätsparameter ein Luftqualitätsparameter ist und der Raum ein Luftraum ist. Alternativ oder zusätzlich kann vorgesehen sein, dass der Qualitätsparameter ein Wasserqualitätsparameter ist und der Raum ein Gewässer ist. Bevorzugt kann vorgesehen sein, dass der Qualitätsparameter ausgewählt ist aus der Gruppe bestehend aus der Konzentration einer Substanz und/oder einer Gruppe von Substanzen, der Absorption und/oder Transmission elektromagnetischer Strahlung durch das Fluid und/oder die Stärke von elektromagnetischer Strahlung an sich. Beispielsweise können unter einem Qualitätsparameter Konzentrationen bestimmter Gase, Flüssigkeiten, oder Feststoffe in der Luft zu verstehen sein. Dabei ist unter dem Messen des Qualitätsparameters auch zu verstehen, dass die Konzentration nicht direkt gemessen wird, sondern ein anderer, messbarer Parameter, beispielsweise eine Absorption, Resistivität, Kapazität oder ähnliches, die als Rohdaten dann in die Konzentration umgerechnet wird. Dabei sind auch die nicht umgerechneten Rohdaten unter Qualitätsparametern zu verstehen. Unter einem Qualitätsparameter kann im Sinne der vorliegenden Erfindung auch ein Parameter zu verstehen sein, der nur mittelbar Einfluss auf die Qualität des Raumes hat. Beispielsweise können auch Konzentrationen von Subtanzen in einem Konzentrationsbereich ein Qualitätsparameter sein, in dem sie nicht unmittelbar Einfluss auf die Qualität des Raumes haben. Somit kann darunter auch das Aufspüren von Substanzen die z.B. zur Vorbereitung/Durchführung eines Terroranschlags dienen, wie Spreng- und Explosivstoffe, chemische, biologische oder radioaktive Substanzen (Kampfstoffe), aber auch Drogen oder Psychopharmaka in geringen Mengen verstanden werden. Unter einer Kontamination ist im Sinne der vorliegenden Erfindung also nicht nur eine Kontamination im Sinne einer gesundheitsgefährdenden Konzentration zu verstehen, sondern eine bestimmbare Abweichung von einem Normalzustand.

Unter einem Istwert ist im Sinne der vorliegenden Erfindung der tatsächlich gemessene Messwert zu verstehen oder der aus den Rohdaten umgerechnete Messwert. Unter einem Sollwert ist im Sinne der vorliegenden Erfindung ein für den Istwert gegebener akzeptabler Wert zu verstehen. Demnach kann der Sollwert durch einen oberen und/oder unteren Grenzwert bestimmt sein. Anwendbare Grenzwerte können beispielsweise ERPG, MAK, IDLH, TEEL, AGW, AEGL, BAT, Explosionsgrenzwerte, Strahlungsgrenzwerte, letale Dosen, oder letale Konzentrationen sein.

Unter einem Messort ist im Sinne der vorliegenden Erfindung der Ort zu verstehen, dessen Fluid gemessen wird. Der Messort kann demnach auch ein korrespondierendes Messvolumen aufweisen.

Unter eine Qualitätsparameter-Istwert ist im Sinne der vorliegenden Erfindung der Istwert des Qualitätsparameters an dem Messort zu verstehen, an dem der Qualitätsparameter gemessen wurde.

Unter einer Mehrzahl von Qualitätsparameter-Istwerten sind demnach im Sinne der vorliegenden Erfindung zumindest zwei Qualitätsparameter-Istwerte zu verstehen. Zumindest zwei Qualitätsparameter-Istwerte können sich im Sinne der vorliegenden Erfindung ausreichend unterscheiden durch den gemessenen Qualitätsparameter an sich und/oder durch den Messort. Eine Mehrzahl von Qualitätsparameter-Istwerten kann beispielsweise durch die Messung eines Qualitätsparameters an zwei verschiedenen Messorten erreicht werden. Alternativ oder zusätzlich kann eine Mehrzahl von Qualitätsparameter-Istwerten durch die Messung verschiedener Qualitätsparameter an einem Messort erreicht werden. Durch das Messen von einem Istwert von zumindest einem Qualitätsparameter an einer Mehrzahl von Messorten wird im Sinne der vorliegenden Erfindung entsprechend bereits eine Mehrzahl von Qualitätsparameter-Istwerten gemessen.

Unter einem mobilen Sensor ist im Sinne der vorliegenden Erfindung ein Sensor zu verstehen, dessen Position in Bezug zum Raum veränderbar ist. Es kann vorgesehen sein, dass der mobile Sensor dabei nicht ständig in Bewegung ist, sondern zumindest temporär auch seine Position halten kann. Unter einem stationären Sensor ist im Sinne der vorliegenden Erfindung ein Sensor zu verstehen, dessen Position im Bezug zum Raum regulär nicht veränderbar ist. Beispielsweise ist darunter ein Sensor zu verstehen, der an einem ortsfesten Gegenstand, wie beispielsweise einer Wand, einem Mast, einem Turm, einem Schornstein oder einer Straßenlaterne befestigt ist. Die Befestigung kann dabei lösbar sein, um beispielsweise eine Wartung oder einen Austausch des stationären Sensors zu ermöglichen.

Demnach wird in dem Verfahren zur Qualitätsüberwachung und Feststellung einer Kontamination eines Raums in Schritt a) eine Mehrzahl von Qualitätsparameter-Istwerten in einem Raum mit einem Sensorsystem gemessen wobei der Raum ein durch Raumkoordinaten bestimmbares Volumen ist, welcher ein Fluid aufweist oder im Wesentlichen mit einem Fluid gefüllt ist. Dabei wird der Istwert von zumindest einem Qualitätsparameter an einer Mehrzahl von Messorten gemessen. An einem Messort wird also jeweils ein Istwert für zumindest einen Qualitätsparameter gemessen, wobei insgesamt jeweils an verschiedenen Messorten ein Istwert gemessen wird und an jedem Messort auch verschiedene Qualitätsparameter gemessen werden können. Das Sensorsystem umfasst eine Mehrzahl von Sensoren, wobei die Sensoren mobile Sensoren sind, oder zumindest ein Sensor ein stationärer Sensor und zumindest ein Sensor ein mobiler Sensor ist. Jeder Sensor misst an einer mittels Raumkoordinaten bestimmten Position des Messortes den zumindest einen Qualitätsparameter-Istwert. Unter einer Mehrzahl von Sensoren sind dabei im Sinne der vorliegenden Erfindung zumindest zwei Sensoren zu verstehen.

Eine Mehrzahl von Sensoren kann beispielsweise ≥ 2 bis ≤ 50, vorzugsweise ≥ 3 bis ≤ 40, weiter bevorzugt ≥ 4 bis ≤ 35, noch weiter bevorzugt ≥ 5 bis ≤ 30, außerdem bevorzugt ≥ 6 bis ≤ 25, weiterhin bevorzugt ≥ 7 bis ≤ 20, mobile Sensoren umfassen. Eine Mehrzahl von Sensoren kann aber auch beispielsweise ≥ 5 bis ≤ 45, vorzugsweise ≥ 8 bis ≤ 42, weiter bevorzugt ≥ 9 bis ≤ 38, noch weiter bevorzugt ≥ 10 bis ≤ 36, außerdem bevorzugt ≥ 12 bis ≤ 32, weiterhin bevorzugt ≥ 15 bis ≤ 18, mobile Sensoren umfassen.

Eine Mehrzahl von Sensoren kann beispielsweise ≥ 1 bis ≤ 50, vorzugsweise ≥ 2 bis ≤ 40, weiter bevorzugt ≥ 4 bis ≤ 35, noch weiter bevorzugt ≥ 5 bis ≤ 30, außerdem bevorzugt ≥ 6 bis ≤ 25, weiterhin bevorzugt ≥ 7 bis ≤ 20, stationäre Sensoren umfassen. Eine Mehrzahl von Sensoren kann aber auch beispielsweise ≥ 3 bis ≤ 45, vorzugsweise ≥ 8 bis ≤ 42, weiter bevorzugt ≥ 9 bis ≤ 38, noch weiter bevorzugt ≥ 10 bis ≤ 36, außerdem bevorzugt ≥ 12 bis ≤ 32, weiterhin bevorzugt ≥ 15 bis ≤ 18, stationäre Sensoren umfassen.

Eine Mehrzahl von Sensoren kann beispielsweise:
- ≥ 2 bis ≤ 50, vorzugsweise ≥ 3 bis ≤ 40, weiter bevorzugt ≥ 4 bis ≤ 35, noch weiter bevorzugt ≥ 5 bis ≤ 30, außerdem bevorzugt ≥ 6 bis ≤ 25, weiterhin bevorzugt ≥ 7 bis ≤ 20, oder ≥ 5 bis ≤ 45, vorzugsweise ≥ 8 bis ≤ 42, weiter bevorzugt ≥ 9 bis ≤ 38, noch weiter bevorzugt ≥ 10 bis ≤ 36, außerdem bevorzugt ≥ 12 bis ≤ 32, weiterhin bevorzugt ≥ 15 bis ≤ 18, mobile Sensoren umfassen; und
- ≥ 1 bis ≤ 20, vorzugsweise ≥ 2 bis ≤ 18, weiter bevorzugt ≥ 4 bis ≤ 16, noch weiter bevorzugt ≥ 5 bis ≤ 14, außerdem bevorzugt ≥ 6 bis ≤ 12, weiterhin bevorzugt ≥ 7 bis ≤ 10 stationäre Sensoren umfassen.

Gemäß einer weiteren Ausführungsform kann das Verhältnis der stationären Sensoren zu den mobilen Sensoren 1 : 3 bis 1 : 100, vorzugsweise 1 : 4 bis 1 : 60, weiter bevorzugt 1 : 5 bis 1 : 50, noch weiter bevorzugt 1 : 10 bis 1 : 30, insbesondere bevorzugt 1 : 15 bis 1 : 20, weiterhin bevorzugt 0 : 200 bis 0 : 20000 ausmachen. Beispielsweise kann ein Schwarm von 100 mobilen Sensoren in ein großes Gebiet von 30 x 100 km, bevorzugt 2 x 4 km, zur Überwachung ausgesendet werden.

In Verfahrensschritt b) werden die an einer Mehrzahl von Messorten gemessenen Qualitätsparameter-Istwerte an ein Datenverarbeitungssystem übertragen. Unter dem Übertragen ist dabei ein systematisches Senden und Empfangen zu verstehen. Es versteht sich, dass die Qualitätsparameter-Istwerte so von dem Sensorsystem gesendet werden, dass sie von dem Datenverarbeitungssystem empfangen, zugeordnet und interpretiert werden können. Das Datenverarbeitungssystem kann aus mehreren Datenverarbeitungssystemen bestehen, die beispielsweise an den mobilen und stationären Sensorträgersystemen, wie Fahrzeug, Luftfahrzeug, Wasserfahrzeug, vorzugsweise autonom, angekoppelt sind oder in einem Datennetz durch systematisches Senden und Empfangen untereinander kommuniziert und in Summe ein Datenverarbeitungssystem ausbilden. Das Datenverarbeitungssystem kann dabei insbesondere die Qualitätsparameter-Istwerte der mittels Raumkoordinaten bestimmten Position des Messortes zuordnen. Dabei kann vorgesehen sein, dass der Sensor die Position des Messortes direkt an das Datenverarbeitungssystem übermittelt. Alternativ kann vorgesehen sein, dass der Sensor seine Identität an das Datenverarbeitungssystem übermittelt und das Steuersystem die dazugehörige Position an das Datenverarbeitungssystem übermittelt. Bei den stationären Sensoren kann vorgesehen sein, dass der Sensor nur seine Identität übermitteln und die Position des Messortes im Datenverarbeitungssystem hinterlegt ist.

In Verfahrensschritt c) werden die an den jeweiligen Messorten gemessenen Qualitätsparameter-Istwerte mit in dem Datenverarbeitungssystem für die jeweiligen Messorte hinterlegten Sollwerten abgeglichen. Es wird somit ermittelt, ob Qualitätsparameter-Istwerte von den Sollwerten abweichen.

In Verfahrensschritt d) wird dann der zumindest eine mobile Sensor mit einem Steuersystem an einen von dem vorherigen Messort des zumindest einen mobilen Sensors beabstandeten Messort bewegt. Somit kann der zumindest eine Sensor nach jedem Verfahrensschritt d) an einem anderen Messort zumindest einen Qualitätsparameter-Istwert messen.

Dabei ist vorgesehen, dass der zumindest eine mobile Sensor verschiedene Messorte nach einem in dem Datenverarbeitungssystem hinterlegten Bewegungsplan ansteuert. Der Bewegungsplan bestimmt also die Bewegung des zumindest einen mobilen Sensors in jedem Verfahrensschritt d). Es kann ein auf den zu überwachenden Raum und an die Anzahl mobiler und die Anzahl stationärer Sensoren angepasster Bewegungsplan hinterlegt sein.

Es ist vorgesehen, dass bei einem Störungsfall, bei dem wenigstens ein Istwert von dem für den jeweiligen Messort definierten Sollwert abweicht, zumindest ein mobiler Sensor mit dem Steuersystem von dem hinterlegten Bewegungsplan abweichend bewegt wird und in dem Raum um die Position des Messortes mit der detektierten Istwertabweichung herum oder vorzugsweise nach Ausbreitungsprognose des Datenverarbeitungssystems um die Konzentrationswolke, zusätzliche Qualitätsparameter-Istwerte an zusätzlichen Messorten misst und an das Datenverarbeitungssystem überträgt, wobei das Datenverarbeitungssystem die räumliche Ausbreitung, Art der Kontamination und/oder Konzentration der jeweiligen Kontamination des Raums bestimmt und vorzugsweise nach Wetterdaten die Ausbreitung erneut prognostiziert. Ein Störungsfall ist demnach ein Fall, in dem in Verfahrensschritt c) eine Abweichung eines Qualitätsparameter-Istwertes von dem Sollwert ermittelt wurde. Somit kann eine Kontamination genau festgestellt werden.

Ein Störungsfall ist dabei ein Fall bei dem wenigstens ein Istwert von dem für den jeweiligen Messort definierten Sollwert abweicht. Der Störungsfall kann dabei nach einer Gefährdung eingestuft werden. Beispielsweise kann ein Störungsfall ein gefährlicher oder ein ungefährlicher Störungsfall sein. Abhängig von der Gefährdungsstufe des Störungsfalls, kann vorgesehen sein, dass lediglich eine Information über die Abweichung durch das Datenverarbeitungssystem erfolgt und eine vom Bewegungsplan abweichende Bewegung von einem mobilen Sensor nicht erfolgt. Beispielsweise kann vorgesehen sein, dass Sandstaub aus der Sahara in Mitteleuropa, Ausbringung von Düngemittel (Mist, Jauche) oder Grasschnitt/Heugeruch nicht zu einer Bewegung im Störungsfall führt.

Durch das vorbeschriebene Verfahren kann vorteilhafter Weise erreicht werden, dass ein Raum mit einer vergleichsweise geringen Anzahl an Sensoren raumdeckend überwacht wird. Durch das vorbeschriebene Verfahren wird dabei vorteilhafter Weise ermöglicht, dass bei einer Überschreitung eines Qualitätsparameters die Überschreitung mit dem System selbst genauer untersucht werden kann, ohne auf eine besonders hohe Anzahl an Sensoren angewiesen zu sein.

Bevorzugt kann vorgesehen sein, dass die Mehrzahl von Sensoren eine Anzahl von stationären Sensoren und eine Anzahl von mobilen Sensoren umfasst, wobei zumindest ein Sensor ein stationärer Sensor ist und zumindest ein Sensor ein mobiler Sensor ist. Dadurch kann vorteilhafterweise durch ein Zusammenspiel von mobilen und stationären Sensoren erreicht werden, durch das das Verfahren besonders flexibel ist und zugleich kosteneffizient.

Bevorzugt kann vorgesehen sein, dass die Mehrzahl von Sensoren zumindest drei Sensoren umfassen. Bevorzugt kann vorgesehen sein, dass die Mehrzahl von Sensoren eine Anzahl von Sensoren umfasst, die an die Anforderungen der Qualitätsüberwachung und Feststellung einer Kontamination des Raums angepasst sind. Beispielsweise kann vorgesehen sein, dass die Mehrzahl von Sensoren zumindest vier, fünf, sechs, sieben, acht, neun oder zehn Sensoren umfasst. Ist der zu überwachende Raum beispielsweise besonders groß oder soll die Qualität besonders dicht überwacht werden, kann vorgesehen sein, dass die Mehrzahl von Sensoren zumindest zwanzig, dreißig, vierzig, fünfzig oder hundert Sensoren umfasst.

Bevorzugt kann vorgesehen sein, dass zumindest zwei Sensoren mobile Sensoren sind. Besonders bevorzugt kann vorgesehen sein, dass zumindest drei, vier, fünf, sechs, sieben, acht, neun oder zehn Sensoren mobile Sensoren sind. Es kann in einer Ausgestaltung vorgesehen sein, dass zumindest zwanzig, dreißig, vierzig, fünfzig oder hundert Sensoren mobile Sensoren sind.

Bevorzugt kann vorgesehen sein, dass zumindest ein Sensor ein stationärer Sensoren ist. Besonders bevorzugt kann vorgesehen sein, dass zumindest zwei, drei, vier, fünf, sechs, sieben, acht, neun oder zehn Sensoren stationäre Sensoren sind. Es kann in einer Ausgestaltung vorgesehen sein, dass zumindest zwanzig, dreißig, vierzig, fünfzig oder hundert Sensoren mobile Sensoren sind.

Die Anzahl mobiler Sensoren kann in einer Ausgestaltung kleiner als die Anzahl stationärer Sensoren sein. Dadurch kann vorteilhafterweise erreicht werden, dass eine grobe Abdeckung des zu überwachenden Raumes besonders einfach und konstant erfolgt. Ferner kann dadurch erreicht werden, dass mit geringem Aufwand bestehende Verfahren zum erfindungsgemäßen Verfahren modifiziert werden können. Die Anzahl mobiler Sensoren kann in einer alternativen Ausgestaltung größer oder gleich der Anzahl stationärer Sensoren sein. Dadurch kann vorteilhafterweise erreicht werden, dass eine besonders flexible Überwachung mit einer besonders geringen Anzahl an Sensoren möglich ist. Dadurch kann beispielsweise die erste Umsetzung des Verfahrens besonders kostengünstig und schnell erfolgen, da nur wenige oder keine stationären Sensoren aufgestellt werden müssen.

In einer Ausgestaltung kann vorgesehen sein, dass der Messort, an dem ein mobiler Sensor ein Qualitätsparameter-Istwert misst, ein Messvolumen aufweist, welches sich entlang eines Bewegungspfads des mobilen Sensor erstreckt. Darunter ist zu verstehen, dass der mobile Sensor während einer Bewegung eine Messung durchführt. Beispielsweise kann der mobile Sensor während der Bewegung für eine bestimmte Zeit einen Messwert ermitteln. Dadurch kann vorteilhafterweise erreicht werden, dass die mobilen Sensoren während der Messung nicht anhalten müssen. Dadurch sind schnellere Messungen möglich. Vorteilhafterweise ergibt sich dadurch auch ein gemittelter Wert für einen größeren Messbereich, so dass weniger Messungen notwendig sein können, um eine gute Abdeckung des Raums zu erhalten.

Bevorzugt kann vorgesehen sein, dass der zumindest eine Qualitätsparameter die Konzentration von Substanzen, insbesondere von Schadstoffen und/oder Gefahrstoffen, in dem Fluid, insbesondere in der Luft ist. Dabei können die Substanzen Gase, Flüssigkeiten oder Feststoffe sein, die in dem Fluid, insbesondere in der Luft, gelöst und/oder dispergiert sind. Der Qualitätsparameter kann somit auch Aerosole oder Rauch betreffen. Die Qualitätsparameter können die Konzentration einer einzelnen Substanz oder einzelner Partikel betreffen oder auch die Konzentration einer Substanzklasse. Der Qualitätsparameter kann auch unabhängig von spezifischen Substanzen einen Qualitätsparameter des Fluids, insbesondere der Luft, beschreiben. Beispielsweise kann die Lichtabsorption der Luft ein Qualitätsparameter sein, ohne spezifisch auf die Konzentration einer Substanz gerichtet zu sein. Der Qualitätsparameter kann dabei jeweils auch der Messwert des Sensors sein.

Es kann vorgesehen sein, dass ein Sensor ein spektroskopischer Sensor ist. Darunter ist zu verstehen, dass der Sensor die Absorption, Emission, Streuung oder Beugung von elektromagnetischer Strahlung misst, beispielsweise von radioaktiver Strahlung, Röntgenstrahlung, ultravioletter Strahlung, sichtbarem Licht, Infrarotstrahlung und/oder Mikrowellenstrahlung. Beispielsweise kann der Sensor ein Raman-, Infrarot- oder UV/Vis-Spektrometer sein. Derartige Sensoren müssen vorteilhafter Weise nicht direkt mit dem Fluid, bzw. der Luft oder dem zu messenden Stoff oder Partikel in Kontakt kommen.

Ferner kann vorgesehen sein, dass ein Sensor ein chemischer Sensor ist. Darunter ist zu verstehen, dass der Sensor zumindest teilweise mit der zu messenden Substanz in Kontakt kommt und durch die chemischen und/oder physikalischen Eigenschaften der Substanz beeinflusst wird. Beispielsweise kann der Widerstand und/oder die Kapazität einer Messschicht eines derartigen Sensors durch die zu messende Substanz beeinflusst werden. Derartige Sensoren können vorteilhafter Weise besonders selektiv für bestimmte Substanzen sein. Besonders vorteilhaft kann ein Sensor ein Metalloxid-Halbleitergassensor sein, ein Festkörper-Ionenleiter und/oder ein Photoionisationsdetektor. Alternativ oder zusätzlich kann vorgesehen sein, dass der Sensor einen Flammenionisationsdetektor, einen Stickstoff-Phosphor Detektor, einen Wärmeleitfähigkeitsdetektor und/oder einen Atomemmisionsdetektor umfasst.

Es kann vorgesehen sein, dass ein Sensor zusätzlich eine Auftrennungsvorrichtung aufweist. Eine Auftrennungsvorrichtung kann dazu dienen das gemessene Fluid, insbesondere die gemessene Luft, vor der Messung eines Qualitätsparameter-Istwertes in Bestandteile aufzutrennen. Beispielsweise kann die Luft über Chromatografie, insbesondere Gaschromatographie aufgetrennt werden. Die Luft kann alternativ oder zusätzlich über ein Ionenmobilitätsspektrometer oder alternativ oder zusätzlich über ein Massenspektrometer, insbesondere über ein Magnetsektorfeld- oder Quadrupol-Massenspektrometer aufgetrennt werden. Somit kann erreicht werden, dass einige Substanzen leichter durch die Sensoren voneinander unterschieden werden können.

Es kann vorgesehen sein, dass der Sensor dazu eingerichtet ist Individuenanalytik zu betreiben, also Qualitätsparameter im Sinne von einzelnen Stoffen zu bestimmen, und/oder dass der Sensor dazu eingerichtet ist Qualitätsparameter im Sinne eines Summenparameters zu bestimmen, wie z.B. TOC, der Gesamtgehalt an organischem Material.

Wenn der Qualitätsparameter organische Flüssigkeiten, die sich verdampfen lassen und organische Gase betrifft, kann besonders bevorzugt vorgesehen sein, dass der Sensor ein Gaschromatograph ist, gekoppelt mit zumindest einem ausgewählt aus der Gruppe von Massenspektrometer (MS und/oder MS/MS), Flammenionisationsdetektor, Stickstoff-Phosphor-Detektor, Wärmeleitfähigkeitsdetektor, Elektroneneinfangdetektor, und Atomemmissionsdetektor. Wenn der Qualitätsparameter Flüssigkeiten betrifft, kann besonders bevorzugt vorgesehen sein, dass der Sensor ein HPLC-MS/MS mit DAD und/oder UV-Vis Detektor ist. Für organische Stoffe, anorganische Verbindungen und/oder Flüssigkeiten kann vorgesehen sein, dass der Sensor ein NMR-Spektrometer, ein Infrarotspektrometer und/oder ein Ramanspektrometer ist. Für organische und anorganische Lösungen und Gase kann bevorzugt vorgesehen sein, dass der Sensor ein UV-Vis-Spektrometer ist. Wenn der Qualitätsparameter Aromaten, Olefine, Bromide und Iodide, Sulfide und Mercaptane, organische Amine, Ketone, Ether, Ester und Acrylate, Aldehyde, Alkohole, Alkane, Schwefelwasserstoff, Ammoniak, und/oder Phosphane betrifft, kann besonders bevorzugt vorgesehen sein, dass der Sensor einen Fotoionisationsdetektor umfasst. Betrifft der Qualitätsparameter organische und anorganische Gase, Sprengstoffe, Drogen, und/oder chemische Kampfstoffe, kann bevorzugt vorgesehen sein, dass der Sensor ein Ionenmobilitätsspektrometer ist. Für anorganische und organische Gase kann bevorzugt vorgesehen sein, dass der Sensor eine elektrochemische Zelle, Prüfröhrchen, thermische Sensoren, Leitfähigkeitssensoren, elektrochemische Diffusionssensoren, und/oder einen Metalloxidsensor umfasst. Zur Messung von Alpha-, Beta- Gamma- und Neutronenstrahlung kann bevorzugt vorgesehen sein, dass der Sensor ein Strahlenmessgerät umfasst. Ferner kann zur Windgeschwindigkeitsmessung und Positionsbestimmung bevorzugt vorgesehen sein, dass der Sensor ein Ultraschall- und/oder Infraschallmessgerät umfasst. Zur Bestimmung von Aerosolen, Staub, Ozon, Stickoxiden, Methan, Schwefeldioxid, Windgeschwindigkeit und/oder zur Objekterkennung/Umgebungserfassung kann bevorzugt vorgesehen sein, dass der Sensor ein LIDAR umfasst. Für Objekterkennung und/oder Umgebungserfassung kann bevorzugt vorgesehen sein, dass der Sensor ein RADAR umfasst. Betrifft der Qualitätsparameter Staub, Biomoleküle und/oder Pollen, kann bevorzugt vorgesehen sein, dass der Sensor ein Partikelzählrohr umfasst. Für Gefahrstoffe und halogenierte Verbindungen, beispielsweise Chlorkohlenwasserstoffe, kann besonders bevorzugt vorgesehen sein, dass der Sensor einen thermisch katalytischen Oberflächenionisationssensor (TKOI) umfasst. Für den Sauerstoffgehalt kann der Sensor bevorzugt ein Ex-Sensor sein. Für die Luftfeuchtigkeit kann der Sensor bevorzugt ein Luftfeuchtigkeitssensor und/oder kapazitiver Sensor sein. Für die Windgeschwindigkeit kann der Sensor bevorzugt ein Windsensor und/oder Anemometer sein. Für Partikel in Flüssigkeiten oder Gasen kann der Sensor ein Turbidimeter und/oder einen optischen Sensor umfassen. Zur Bestimmung des Brechungsindexes von Flüssigkeiten oder Gasen kann der Sensor ein Refraktometer umfassen. Ferner kann der Sensor zur Bestimmung der Temperatur ein Thermometer umfassen. Zur Bestimmung des Luftdruckes kann der Sensor einen Drucksensor umfassen. Zur Bestimmung des pH-Wertes kann der Sensor ferner bevorzugt ein pH-Meter umfassen. Zur Bestimmung des Salzgehaltes kann der Sensor bevorzugt ein Leitfähigkeitsmessgerät umfassen. Betrifft der Qualitätsparameter biologische Aerosolpartikel und/oder Sporen, kann der Sensor bevorzugt ein Fluoreszenz-Messgerät umfassen. Betrifft der Qualitätsparameter organische Gase, kann der Sensor bevorzugt einen VOC-Sensor umfassen. Betrifft der Qualitätsparameter anorganische Gase, beispielsweise Li, Na, K, Ca etc., kann der Sensor bevorzugt ein Flammenphotometer umfassen. Betrifft der Qualitätsparameter Kohlenstoffdioxid und/oder andere IR-aktive Gase, kann bevorzugt vorgesehen sein, dass der Sensor einen Festkörper-Gassensor (NDIR) umfasst.

Es kann außerdem vorgesehen sein, dass ein Sensor ein optischer Bildsensor ist. Somit kann zusätzlich auch ein Bild des Raumes aufgenommen werden. Der Bildsensor kann dabei auch kontinuierlich Bilder des Raumes und insbesondere des Messortes aufnehmen.

Es kann vorgesehen sein, dass mit einem Sensor verschiedene Qualitätsparameter an einem Messort gemessen werden. Darunter ist zu verstehen, dass der Sensor dazu eingerichtet sein kann unterschiedliche Qualitätsparameter zu messen. Dafür kann vorgesehen sein, dass der Sensor bevorzugt verschiedene Messysteme umfasst, wobei jedes Messystem dazu eingerichtet ist zumindest einen Qualitätsparameter zu messen. Beispielsweise kann der Sensor ein spektroskopischer Sensor und/oder ein chemischer Sensor sein. Darunter ist zu verstehen, dass der Sensor ein Messystem aufweist, das dazu eingerichtet ist spektroskopische Messungen durchzuführen, und ein Messystem, das dazu eingerichtet ist chemische Messungen durchzuführen.

Nach einer bevorzugten Ausgestaltung kann vorgesehen sein, dass das Sensorsystem zusätzliche mobile Sensoren aufweist, die nicht einem Bewegungsplan folgen und nicht durch das Steuersystem gesteuert werden. Beispielsweise kann das Sensorsystem Sensoren umfassen, die von Personen getragen werden, beispielsweise Personendosimeter oder Sensoren zur Arbeitsplatzüberwachung, beispielsweise Gaswarngeräte, oder Sensoren, die an Fahrzeugen befestigt sind, die nicht durch das Steuersystem gesteuert werden. Diese Sensoren können mit einer Positionsbestimmungvorrichtung ausgestattet sein und neben Messwerten auch den dazugehörigen Messort angeben.

Nach einer bevorzugten Ausgestaltung kann vorgesehen sein, dass das Messen eines Qualitätsparameter-Istwertes eine Probennahme umfasst. Unter einer Probennahme ist dabei das Einsammeln einer Probe zu verstehen, insbesondere das Einsammeln und Mitnehmen einer Probe. Dabei kann vorgesehen sein, dass die Probennahme mit einem Passivsammler durchgeführt wird, der aufgrund der längeren Expositionszeit die Nachweisgrenze des Systems herabsetzen kann. Dabei kann vorgesehen sein, dass der Passivsammler stationär ist und von einem mobilen Sensor aufgesammelt, gemessen und ausgewertet wird. Das stationäre System kann dann durch die mobilen Sensoren mit einem neuen oder dem "Rückgesetzten" Passivsammler ausgestattet werden. Es kann nach einer bevorzugten Ausgestaltung der Erfindung vorgesehen sein, dass der mobile Sensor eine Probe nimmt und zur Analyse einem anderen Sensor übergibt. Beispielsweise kann vorgesehen sein, dass ein fliegender mobiler Sensor eine Probe nimmt und an einen auf dem Boden fahrenden Sensor oder einen stationären Sensor übergibt. Der auf dem Boden fahrende Sensor oder stationäre Sensor kann dann die Probe analysieren und den Qualitätsparameter-Istwert an das Datenverarbeitungssystem übermitteln. Dadurch können bevorzugt auch komplizierte Messmethoden verwendet werden. Insbesondere können die mobilen Sensoren dadurch klein gehalten werden. Es kann zudem erreicht werden, dass die Anzahl von insbesondere besonders teuren Sensoren reduziert werden kann, da mehrere mobile Sensoren ihre Fluidproben bei einem einzigen Sensor abgeben können.

Bevorzugt kann vorgesehen sein, dass die Probennahme mit einem Sammelsystem durchgeführt wird, ausgewählt aus der Gruppe bestehend aus solid phase microextraction Fasern, "Gasmäuse", Aktivkohle-Röhrchen, Tenax^{®}-Röhrchen, Reservoir-Gefäße für Gase und/oder Flüssigkeiten.

Probennahmen können dabei bevorzugt veranlasst werden, bei der Detektion unbekannter Stoffen, bzw. bei einer Erhöhung bestimmter Qualitätsparameter-Istwerte, die ohne zusätzliche Messung keiner Substanz zugeordnet werden können. Alternativ oder zusätzlich kann eine permanente Probenahme mit Passivsammlern vorgesehen sein, die in regelmäßigen Abständen eingesammelt/vermessen werden. Es kann vorteilhafter Weise vorgesehen sein, die Proben durch die mobile Sensoreinheiten zu messen. Alternativ oder zusätzlich kann vorgesehen sein, die Proben mit den mobilen Sensoren zum Ort der Analyse, z.B. außerhalb von Gefahrenbereichen, zu transportieren. Der Transportweg durch die Luft mittels der mobilen Sensoren kann dabei deutlich die Zeit bis zum Erhalt der Ergebnisse reduzieren. In einer besonders bevorzugten Ausgestaltung kann vorgesehen sein, dass der Transport zu einem zentralen und/oder spezialisierten Sensor durchgeführt wird. Besonders bevorzugt können Passivsammler an Stelle von Sensoren an Orten mit niedriger Eintrittswahrscheinlichkeit verbaut werden.

Nach einer bevorzugten Ausgestaltung kann vorgesehen sein, dass die Probennahme auch ein Sammeln von Rückstellproben umfasst. Besonders bevorzugt kann vorgesehen sein, dass die mobilen Sensoren die Proben nehmen und an ein Lagersystem übergeben. Dadurch kann gegebenenfalls der Nachweis über die Qualität des Raumes, insbesondere eines Luftraumes, geführt werden.

Der Bewegungsplan kann dabei für jeden mobilen Sensor eine Abfolge von Messorten vorsehen. Die Messorte können vorteilhafter weise so gewählt sein, dass sie den Raum möglichst homogen abdecken. Die Messorte können dabei in bestimmten Bereichen des Raums enger zueinander gewählt werden, beispielsweise Gefahrenquellen. Der Bewegungsplan kann auch ein Zeitplan sein, so dass der mobile Sensor in einem bestimmten zeitlichen Abstand an die verschiedenen Messorte bewegt wird. Es kann vorgesehen sein, dass der Bewegungsplan auch einen Zeitplan nach bestimmten Tageszeiten vorsieht. Beispielsweise können bestimmte Bereiche des Raums zu bestimmten Tageszeiten geringer beabstandete Messorte als zu anderen Tageszeiten vorsehen. Zudem kann der Bewegungsplan die Anzahl mobiler Sensoren und/oder die Anzahl stationärer Sensoren berücksichtigen. Der Bewegungsplan kann für jeden mobilen Sensor dieselben Messorte vorsehen, wobei die mobilen Sensoren zu den vorgesehenen Messorten zeitlich versetzt bewegt werden. Alternativ kann der Bewegungsplan für verschiedene mobile Sensoren unterschiedliche Messorte vorsehen. Beispielsweise kann der Bewegungsplan für jeden mobilen Sensor einen eigenen Bewegungsplan vorsehen.

Der Bewegungsplan kann somit besonders gut an die gegebenen Bedingungen des Raumes und das Sensorsystem angepasst sein. Somit kann besonders einfach eine effiziente Überwachung des Raums erreicht werden. Die Überwachung kann zudem dadurch besonders einfach an Veränderungen des Sensorsystems, des Raums und/oder der Umgebung des Raums angepasst werden.

Im Störungsfall weicht zumindest ein mobiler Sensor mit dem Steuersystem von dem hinterlegten Bewegungsplan ab. Es kann insbesondere vorgesehen sein, dass der mobile Sensor, der im Störungsfall am wenigsten von dem für den jeweiligen Messort definierten Sollwert abweichenden Istwert beabstandet ist um die Position des Messortes mit der detektierten Istwertabweichung herum bewegt wird. In einer bevorzugten Ausgestaltung kann vorgesehen sein, dass mehrere oder alle mobilen Sensoren um die Position des Messortes mit der detektierten Istwertabweichung bewegt werden. Dem Störungsfall kann dabei nach in Abhängigkeit der Art des Qualitätsparameters und der Größe der Abweichung eine Dringlichkeit zugeordnet werden. In Abhängigkeit der Dringlichkeit kann dann eine Anzahl eingesetzter mobiler Sensoren ausgewählt werden, wobei bei einem Störungsfall mit höchster Dringlichkeit alle mobilen Sensoren eingesetzt werden können und bei einem Störungsfall mit niedrigster Dringlichkeit zumindest ein mobiler Sensor eingesetzt wird. Das Sensorsystem kann zudem zusätzliche mobile Sensoren aufweisen, die gerade nicht dem Bewegungsplan gefolgt sind, beispielsweise Sensoren, die gerade auf Abruf bereitstehen. In einem Störungsfall können auch diese zusätzlichen mobilen Sensoren eingesetzt werden. Zusätzlich kann im Störungsfall vorgesehen sein, dass ein stationärer Sensor seine Messfrequenz erhöht. Beispielsweise kann vorgesehen sein, dass ein stationärer Sensor, der in einem bestimmten Zeitintervall Messungen durchführt im Störungsfall in einem kleineren Zeitintervall Messungen durchführt. Es kann auch vorgesehen sein, dass ein stationärer Sensor in einem Stand-by-Modus ist und erst im Störungsfall Messungen durchführt.

Dadurch kann vorteilhafter Weise erreicht werden, dass die Kontamination des Raums in einem Störungsfall genau festgestellt werden kann. Dadurch, dass die bestehenden mobilen Sensoren verwendet werden, kann einerseits sehr schnell auf eine Kontamination des Raums reagiert werden und es können Ressourcen gespart werden, da auf zusätzliche Notfallsysteme zur Feststellung der Kontamination verzichtet werden kann.

Die Bewegung um die Position des Messortes mit der detektierten Abweichung kann dabei in einem Schritt nach einem Lokalisierungsplan erfolgen. Dabei können die eingesetzten mobilen Sensoren systematisch um den Messort mit der detektierten Abweichung bewegt werden. In einem anderen Schritt kann die Bewegung um die Position des Messortes entsprechend der dabei gemessenen Qualitätsparameter-Istwerte angepasst werden. Beispielsweise können die Sensoren entlang steigender Qualitätsparameter-Istwerte bewegt werden oder entlang gleichbleibender oder fallender Qualitätsparameter-Istwerte.

Somit kann erreicht werden, dass die genaue Ausbreitung der Kontamination bestimmt werden kann. Zudem kann der Verlauf der Kontamination über die Zeit bestimmt werden und es kann gegebenenfalls die Quelle der Kontamination bestimmt werden.

Nach einer bevorzugten Ausgestaltung des Verfahrens kann vorgesehen sein, dass in Schritt a) zusätzlich weitere Fluidparameter, insbesondere Luftparameter, insbesondere Windrichtung, Windstärke, Lufttemperatur, Luftfeuchtigkeit und/oder Luftdruck gemessen werden, und in Schritt b) an das Datenverarbeitungssystem übertragen werden. Bevorzugt kann vorgesehen sein, dass der Bewegungsplan an die weiteren Fluidparameter, insbesondere Luftparameter, angepasst wird. Beispielsweise kann vorgesehen sein, dass entsprechend einer gemessenen Windrichtung und Windstärke die Messorte des Bewegungsplans verschoben werden.

Dadurch kann erreicht werden, dass die Messorte besonders effizient ausgewählt werden können.

Somit kann eine besonders effiziente Luftüberwachung erreicht werden.

Nach einer bevorzugten Ausgestaltung kann vorgesehen sein, dass der zumindest eine Qualitätsparameter-Istwert die Konzentration einer Substanz in dem Fluid, insbesondere in der Luft ist, insbesondere die Konzentration eines Gefahrstoffes und/oder Schadstoffes.

Beispielsweise kann der Qualitätsparameter-Istwert die Konzentration in der Luft sein von Ammoniak, Flüchtige organische Verbindungen ohne Methan (NMVOC), Kohlenstoffmonoxid, Schwefeldioxid, Staub (unter besonderer Berücksichtigung der Fraktionen PM10 und PM2,5), Stickoxiden, Persistente organische Schadstoffe (POP) und Schwermetalle. Der Qualitätsparameter-Istwert kann auch die Konzentration von einer in der Umgebung des Messwertes häufiger vorkommenden Substanz sein. Beispielsweise kann der Qualitätsparameter-Istwert um entsprechende Industrieanlagen die Konzentration der in der Industrieanlage verwendeten Stoffe, insbesondere der durch die Industrieanlage möglicherweise ausgestoßenen Abgase, Emissionen und/oder Gefahrstoffe, sein.

Nach einer bevorzugten Ausgestaltung kann vorgesehen sein, dass dem Datenverarbeitungssystem weitere Umgebungsdaten bereitgestellt werden, insbesondere Informationen über mögliche Emissionsquellen.

Dadurch kann erreicht werden, dass der Bewegungsplan entsprechend angepasst werden kann. Zudem können die Umgebungsdaten in einem Störungsfall verwendet werden um zielgerichteter nach einer Ursache für die Sollwertabweichung zu suchen.

Nach einer bevorzugten Ausgestaltung kann vorgesehen sein, dass die weiteren Umgebungsdaten dem Datenverarbeitungssystem automatisch, semi-automatisch und/oder manuell bereitgestellt werden. Automatisch bereitgestellte weitere Umgebungsdaten können dabei beispielsweise von externen Quellen dem Datenverarbeitungssystem kontinuierlich zur Verfügung gestellt werden. Beispielsweise kann vorgesehen sein, dass dem Datenverarbeitungssystem Informationen über das Wetter oder über die umgebende Verkehrslage kontinuierlich zur Verfügung gestellt werden. Semi-automatisch bereitgestellte weitere Umgebungsdaten können Informationen sein, die nur in bestimmten Fällen automatisch oder manuell ausgelöst dem Datenverarbeitungssystem zur Verfügung gestellt werden. Beispielsweise können Betriebsstörungen automatisch oder manuell an das Datenverarbeitungssystem übermittelt werden. Manuell bereitgestellte weitere Umgebungsdaten können bevorzugt weitere Umgebungsdaten sein, die nicht zuvor klassifiziert sind. Beispielsweise können Beobachtungen der Bevölkerung dem Datenverarbeitungssystem bereitgestellt werden. Beispielsweise können Gerüche oder Rauch von Bewohnern wahrgenommen werden und dem Datenverarbeitungssystem durch Telefonanrufe in einer Zentrale mitgeteilt werden. Dafür ist vorgesehen, dass das Datenverarbeitungssystem eine Eingabeschnittstelle aufweist, über die dem Datenverarbeitungssystem Informationen über weitere Umgebungsdaten auch manuell mitgeteilt werden können.

Die bereitgestellten Umgebungsdaten können dabei auch dynamische Umgebungsdaten, insbesondere in Echtzeit, sein. Beispielsweise können Daten über Straßen-, Schiffs- oder Flugverkehr bereitgestellt werden. Unter dynamischen Umgebungsdaten sind dabei insbesondere Informationen über mögliche Emissionsquellen zu verstehen, die veränderbar sind. Beispielsweise können darunter Informationen über natürliche oder technische Ereignisse zu verstehen sein, die Einfluss auf die Qualität haben. Beispielsweise können Informationen über Verkehrsstaus von naheliegenden Straßen, Straßenmarkierungsarbeiten, Baustellen, Vulkanausbrüche und/oder Sandstürme dynamische Umgebungsdaten sein. Alternativ oder zusätzlich können beispielsweise Daten über Produktionspläne von entsprechenden Industrieanlagen bereitgestellt werden. Dabei können die Informationen über möglicherweise emittierte Substanzen enthalten. Somit kann der Bewegungsplan entsprechend der Umgebungsdaten verändert werden. Beispielsweise können mobile Sensoren, zu überwachende Qualitätsparameter und Messorte entsprechend der zu erwartenden Art und Position der Emissionen ausgewählt werden.

Nach einer bevorzugten Ausgestaltung kann vorgesehen sein, dass der Bewegungsplan ortsfeste Objekte mit ihren Raumkoordinaten aufweist zu denen ein Minimalabstand und/oder Maximalabstand für die Annäherung des mobilen Sensors hinterlegt ist. Darunter ist zu verstehen, dass der Bewegungsplan Bewegungskorridore vorsehen kann oder Bereiche, in die der mobile Sensor auf keinen Fall bewegt werden soll bzw. darf.

Dadurch kann insbesondere erreicht werden, dass die Bewegung des mobilen Sensors durch das Steuersystem vereinfacht werden kann. Insbesondere können ortsfeste Objekte berücksichtigt werden, so dass Kollisionen mit diesen Objekten ohne großen Aufwand verhindert werden können. Zudem kann verhindert werden, dass nicht ausreichend geschützte mobile Sensoren beispielsweise in explosionsgefährdete Bereiche bewegt werden. Somit kann die Sicherheit des Verfahrens erhöht werden.

Nach einer bevorzugten Ausgestaltung kann vorgesehen sein, dass im Bewegungsplan Messorte definiert sind, die wenigstens ein mobiler Sensor in einer vorgegebenen Reihenfolge oder statistisch ansteuert; und/oder wenigstens ein mobiler Sensor/en die Messorte innerhalb des zu überwachenden Raums zufällig auswählt.

Durch eine vorgegebene Reihenfolge kann erreicht werden, dass eine Mindestüberwachung einfach gewährleistet werden kann. Durch zufällig ausgewählte Messorte kann erreicht werden, dass statistische Fehler minimiert werden. Insbesondere kann vorgesehen sein, dass bei einer Mehrzahl mobiler Sensoren einige mobile Sensoren definierte Messorte in einer vorgegebenen Reihenfolge ansteuern und andere mobile Sensoren Messorte innerhalb des zu überwachenden Raums zufällig auswählen.

Es kann vorgesehen sein, dass bei einer Mehrzahl mobiler Sensoren, der Bewegungsplan eine möglichst große räumlich Verteilung der mobilen Sensoren vorsieht. Darunter ist zu verstehen, dass die Sensoren besonders gleichmäßig über den zu überwachenden Raum verteilt sind.

Es kann nach einer bevorzugten Ausgestaltung vorgesehen sein, dass der Bewegungsplan dynamisch angepasst wird. Dadurch kann erreicht werden, dass in jeder gegebenen Lage der Bewegungsplan eine optimale Überwachung der Qualitätsparameter erlaubt. Das dynamische Anpassen des Bewegungsplans kann dabei in einer bevorzugten Ausgestaltung ein Anpassen an die Umgebungsdaten sein. Dabei kann vorgesehen sein, dass der Bewegungsplan an manchen Orten im Raum eine größere Messhäufigkeit oder eine größere Messdichte vorsieht. Beispielsweise kann vorgesehen sein, dass der Bewegungsplan an Orten mit einer größeren Wahrscheinlichkeit für einen Störungsfall eine größere Messhäufigkeit vorsieht. Es kann bevorzugt auch vorgesehen sein, dass der Bewegungsplan an Orten bei denen von einem potentiellen Störungsfall eine größere Gefahr ausgeht eine größere Messhäufigkeit vorsieht. Zudem kann vorgesehen sein, dass ein Anpassen des Bewegungsplanes an die Umgebungsdaten ein Anpassen an die Windrichtung und/oder das Wetter umfasst. Das Anpassen kann auch ein Anpassen an die Tageszeit, den Wochentag, den Monat und/oder die Jahreszeit sein.

Es kann nach einer bevorzugten Ausgestaltung vorgesehen sein, dass das Anpassen des Bewegungsplans auf Basis historischer Messdaten des Verfahrens und Umgebungsdaten erfolgt. Bevorzugt kann dabei eine künstliche Intelligenz eingesetzt werden, die dazu eingerichtet ist den Bewegungsplan, aus historischen Messdaten und Umgebungsdaten anzupassen, um eine besonders effiziente Überwachung des Raums zu erhalten. Es kann beispielsweise vorgesehen sein, dass Bereiche identifiziert werden, die ein besonders hohes Risiko bei einer Störung oder eine besonders hohe Störhäufigkeit aufweisen, und der Bewegungsplan automatisch modifiziert wird um eine zeitlich und örtlich besonders dichte Überwachung dieser Bereiche zu erhalten. Dadurch kann erreicht werden, dass das Verfahren sich über die Verwendungsdauer immer besser an die Gegebenheiten des Raums anpasst und auch auf Veränderungen reagieren kann. Beispielsweise kann der Bewegungsplan so automatisch an neue Gefahrenquellen, wie beispielsweise neue oder gealterte Produktionsanlagen, angepasst werden.

Nach einer bevorzugten Ausgestaltung kann vorgesehen sein, dass wenigstens ein mobiler Sensor im Störungsfall den Ort der höchsten Konzentration der über dem Sollwert liegenden Substanz in dem Fluid, insbesondere in der Luft ermittelt.

Dadurch kann insbesondere festgestellt werden, ob die Emission weiter persistiert. Zudem kann so verfolgt werden, an welchem Ort die größte Gefahr vorliegt und in welche Richtung sich dieser Ort möglicherweise bewegt. Somit können entsprechende Sicherungsmaßnahmen besonders zielgerichtet erfolgen.

Nach einer bevorzugten Ausgestaltung kann vorgesehen sein, dass die von dem/den stationären Sensor/en und/oder mobilen Sensor/en an das Datenverarbeitungssystem übermittelten Qualitätsparameter-Istwerte vom Datenverarbeitungssystem zur Erstellung einer Falschfarbenkarte unter Berücksichtigung der Sollwerte verarbeitet werden.

Dadurch kann erreicht werden, dass die durch die Luftüberwachung gewonnenen Informationen gut verstanden, berichtet und weiterverarbeitet werden können.

Nach einer bevorzugten Ausgestaltung kann vorgesehen sein, dass das Datenverarbeitungssystem auf Basis von zumindest einem Qualitätsparameter-Istwert und weiterer Luftdaten den Qualitätsparameter-Istwert zeitlich und/oder örtlich extrapoliert.

Dadurch kann erreicht werden, dass die Quelle für eine Emission einfach gefunden werden kann. Zudem kann dadurch erreicht werden, dass Schutzmaßnahmen besonders vorausschauend und schnell eingeleitet werden können. Besonders bevorzugt kann vorgesehen sein, dass das System selbst die Schutzmaßnahmen einleitet.

Nach einer bevorzugten Ausgestaltung kann vorgesehen sein, dass im Störungsfall eine Markersubstanz ausgebracht wird. Unter einer Markersubstanz ist dabei eine im Wesentlichen ungefährliche Substanz zu verstehen, die einfach durch Sensoren qualitativ und vorzugsweise auch quantitativ gemessen werden kann. Die Markersubstanz kann dann im Störungsfall wie ein Qualitätsparameter-Istwert gemessen werden. Dadurch kann erreicht werden, dass auch Sensoren, die nicht zum Messen des überschrittenen Qualitätsparameter-Istwertes ausgestattet sind, eine Ausbreitung über die Markersubstanz verfolgen können. Dabei kann die Markersubstanz beispielsweise auch für Menschen wahrnehmbar, insbesondere sichtbar sein.

Nach einer bevorzugten Ausgestaltung kann vorgesehen sein, dass das Datenverarbeitungssystem bei einer Überschreitung des Ist-Wertes vom Soll-Wert und abhängig von der Ausbreitung und Art der Überschreitung des Ist-Wertes vom Soll-Wert automatische Warnungen ausgibt, wobei das Datenverarbeitungssystem wieder Entwarnung gibt, wenn die Qualitätsparameter-Istwert/e den im Datenverarbeitungssystem, für den jeweils mittels stationärem Sensor/en und/oder mobilem Sensor/en untersuchten Raum, hinterlegten Sollwert/en, entsprechen.

Beispielsweise kann vorgesehen sein, dass das Datenverarbeitungssystem Warnungen an Warn-Apps weiterleiten. Zudem kann das Datenverarbeitungssystem Hilfsdienste wie die Feuerwehr oder den Katastrophenschutz, oder auch die zuständigen privaten Sicherheitsdienste informieren.

Dadurch kann erreicht werden, dass gegebenenfalls Hilfe schneller bereitgestellt werden kann.

Nach einer bevorzugten Ausgestaltung kann vorgesehen sein, dass das Datenverarbeitungssystem bei einer Überschreitung des Ist-Wertes vom Soll-Wert entsprechend eines diesbezüglich im Datenverarbeitungssystem hinterlegten Maßnahmenplans Gefahrenabwehrmaßnahmen vorschlägt und/oder durchführt, insbesondere ein Abschalten möglicher Emissionsquellen und/oder eine Absperrung von Gefahrenbereichen.

Dies ist insbesondere für besonders gefährliche und einfach zu steuernde Quellen vorgesehen. Beispielsweise könnte im Datenverarbeitungssystem hinterlegt sein, dass eine bestimmte Produktionsanlage bei Fehlfunktion spezifische Schadstoffe ausstoßen kann. Im hinterlegten Maßnahmenplan kann hinterlegt sein, dass bei einer Überschreitung des Sollwertes des spezifischen Schadstoffes die Produktionsanlage heruntergefahren wird.

Weitere Maßnahmen können sein, der Verschluss von Lüftungsöffnungen, das Einschalten von Abluftanlagen, das Auslösen von Warnanlagen, die Betätigung, insbesondere das Schließen, von Druckentlastungseinrichtungen, Entspannungseinrichtungen, Notventilen und Notklappen. Dadurch kann erreicht werden, dass besonders gefährliche Emissionen schnellstmöglich gestoppt werden.

Nach einer bevorzugten Ausgestaltung kann vorgesehen sein, dass der mobile Sensor abhängig vom jeweiligen Messort und auf Basis des gemessenen Qualitätsparameter-Istwert, diese Daten an das Datenverarbeitungssystem zur Erstellung eines Sollwert-Profils für unterschiedliche Messorte des Raums, sendet.

Darunter ist zu verstehen, dass ein Sollwert auch unter einem üblichen Grenzwert liegen kann. Somit kann durch das Verfahren eine Abweichung schneller erkannt werden, ohne die Anzahl von Fehlalarmen übermäßig zu erhöhen. Insbesondere kann durch Erstellung eines Sollwert-Profils erreicht werden, dass bereits sehr kleine Abweichungen vom Sollwert-Profil gemessen werden können. Derartigen Abweichungen kann dann beispielsweise gesondert nachgegangen werden. So können bereits kleine Abweichungen Hinweise geben auf Materialermüdungen oder bevorstehende Emissionen. Es kann somit vorgesehen sein, dass präventive Maßnahmen bei Abweichungen vom Sollwert-Profil eingeleitet werden.

Mit der Erfindung wird ferner ein System zur Qualitätsüberwachung und Feststellung einer Kontamination eines Raums vorgeschlagen wobei der Raum ein durch Raumkoordinaten bestimmbares Volumen ist, welcher ein Fluid aufweist oder der im Wesentlichen mit einem Fluid gefüllt ist.

Das System weist auf, ein Sensorsystem, ein Datenverarbeitungssystem und ein Steuersystem, wobei das Sensorsystem eine Mehrzahl von Sensoren aufweist, wobei die Sensoren mobile Sensoren sind, oder zumindest ein Sensor ein stationärer Sensor und zumindest ein Sensor ein mobiler Sensor ist, wobei das Sensorsystem dazu eingerichtet ist eine Mehrzahl von Qualitätsparameter-Istwerten in einem Raum zu messen, wobei der Raum ein durch Raumkoordinaten bestimmbares Volumen ist, welcher ein Fluid aufweist oder der im Wesentlichen mit einem Fluid gefüllt ist, wobei der Istwert von zumindest einem Qualitätsparameter an einer Mehrzahl von Messorten gemessen wird, wobei jeder Sensor an einer mittels Raumkoordinaten bestimmten Position des Messortes den zumindest einen Qualitätsparameter-Istwert misst, wobei das Sensorsystem dazu eingerichtet ist, die an einer Mehrzahl von Messorten gemessenen Qualitätsparameter-Istwerte an das Datenverarbeitungssystem zu übertragen, wobei das Datenverarbeitungssystem dazu eingerichtet ist, die an einer Mehrzahl von Messorten gemessenen Qualitätsparameter-Istwerte mit für den jeweiligen Messort bereitgestellten Sollwerten zu vergleichen, wobei das Steuersystem dazu eingerichtet ist, den zumindest einen mobilen Sensor an einen von dem vorherigen Messort des zumindest einen mobilen Sensors beabstandeten Messort zu bewegen, wobei der zumindest eine mobile Sensor verschiedene Messorte nach einem in dem Datenverarbeitungssystem hinterlegten Bewegungsplan ansteuern, dadurch gekennzeichnet, dass bei einem Störungsfall, bei dem wenigstens ein Istwert von dem für den jeweiligen Messort definierten Sollwert abweicht, zumindest ein mobiler Sensor mit dem Steuersystem von dem hinterlegten Bewegungsplan abweichend bewegt wird und in dem Raum um die Position des Messortes mit der detektierten Istwertabweichung herum, zusätzliche Qualitätsparameter-Istwerte an zusätzlichen Messorten misst und an das Datenverarbeitungssystem überträgt, wobei das Datenverarbeitungssystem die räumliche Ausbreitung, Art der Kontamination und/oder Konzentration der jeweiligen Kontamination des Raums bestimmt.

Durch das vorbeschriebene System kann vorteilhafter Weise ein Raum besonders effizient überwacht werden und eine Kontamination des Raums besonders präzise und schnell festgestellt werden. Das System dient somit der Ausführung des vorbeschriebenen Verfahrens.

Nach einer bevorzugten Ausgestaltung kann vorgesehen sein, dass das Datenverarbeitungssystem Rechner bzw. Datenverarbeitungseinrichtungen aufweist, die miteinander kommunizieren können, wobei vorgesehen sein kann, dass ein Rechner bzw. Datenverarbeitungseinrichtung an dem zumindest einen mobile Sensor angeordnet ist. Das Datenverarbeitungssystem kann aus mehreren Datenverarbeitungssystemen bestehen, die beispielsweise an den mobilen und stationären Sensorträgersystemen, wie Fahrzeug, Luftfahrzeug, Wasserfahrzeug, vorzugsweise autonom, angekoppelt sind oder in einem Datennetz durch systematisches Senden und Empfangen untereinander kommuniziert/en und in Summe ein effizienteres Datenverarbeitungssystem ausbildet.

Nach einer bevorzugten Ausgestaltung kann vorgesehen sein, dass der mobil angeordnete Sensor ein Bodenfahrzeug, Schienenfahrzeug, Seilbahnfahrzeug, Wasserfahrzeug und/oder ein Luftfahrzeug ist, wobei das System insbesondere zumindest zwei mobil angeordnete Sensoren aufweist und ein mobil angeordneter Sensor ein Bodenfahrzeug, Schienenfahrzeug, Seilbahnfahrzeug oder Wasserfahrzeug und ein anderer mobil angeordneter Sensor ein Luftfahrzeug, insbesondere Drohne, ist. Darunter, dass ein Sensor ein bestimmtes Fahrzeug ist, ist zu verstehen, dass ein entsprechender Sensor an einem entsprechenden Fahrzeug angeordnet ist. Im Sinne der vorliegenden Erfindung ist ein mobiler Sensor also auch ein einen fest angeordneten Sensor aufweisendes Fahrzeug.

Durch Bodenfahrzeuge kann das System besonders einfach umgesetzt werden. Bodenfahrzeuge sind an sich bekannt und besonders sicher, da sie bei Fehlfunktionen zumeist einfach stehen bleiben. Zur raumdeckenden Luftüberwachung ist es jedoch wünschenswert, dass Messorte auch deutlich oberhalb des Bodens angeordnet sind. Es kann somit vorgesehen sein, dass das Bodenfahrzeug Aufbauten aufweist, die dazu ausgestaltet sind, Messorte in verschiedenen Höhen zu erreichen, beispielsweise Teleskoparme.

Mobile Sensoren können auch Fahrzeuge umfassen. Vorzugsweise sind diese Fahrzeuge mit einem Navigationssystem ausgestattet, welches dem Fahrer im Fall eines Ereignisses Fahranweisungen erteilt, um erneute Messungen in sicherer Entfernung durchzuführen oder sich aus dem Gefahrenbereich zu entfernen. Vorzugsweise auch autonome Fahrzeuge, die im Fall eines Ereignisses in den Gefahrenbereich gesteuert werden können und Messwerte ermitteln, um eine verbesserte Ausbreitungsprognose zu erstellen und über die Gefahrenzonen im Lagebild genauer zu informieren.

Durch Luftfahrzeuge können Messorte in verschiedenen Höhen besonders gut erreicht werden. Besonders bevorzugt sind die Luftfahrzeuge Multicopter und/oder Luftschiffe, beispielsweise mit angehängten Sensoren, optional am Seil in unterschiedlichen Höhen. Derartige Luftfahrzeuge können vorteilhafter Weise einfach gesteuert werden und insbesondere in der Luft stehen. Dadurch kann vorteilhafter Weise erreicht werden, dass nahezu an beliebigen Messorten und Messhöhen gemessen werden kann.

Durch Wasserfahrzeuge können Messorte in verschiedenen Tiefen in Gewässern und Meeren besonders gut erreicht werden und über lange Zeit mit Energie versorgt werden. Besonders bevorzugt sind die Wasserfahrzeuge mit angehängten Sensoren beispielsweise am Seil in unterschiedlichen Tiefen. Derartige Wasserfahrzeuge können vorteilhafter Weise einfach gesteuert werden und insbesondere in dem Gewässer stehen. Dadurch kann vorteilhafter Weise erreicht werden, dass nahezu an beliebigen Messorten und Messtiefen gemessen werden kann, beispielsweise Sauerstoffgehalt, CO2-Konzentration etc..

Nach einer bevorzugten Ausgestaltung kann vorgesehen sein, dass das Bodenfahrzeug, Schienenfahrzeug, Seilbahnfahrzeug, Wasserfahrzeug und/oder das Luftfahrzeug autonom betreibbar ist, insbesondere in den Autonomiestufen 4 oder 5. Beispielsweise können die Fahrzeuge UAV's, UGV's, UWV's, UUV's, AUV's oder USV's sein.

Dadurch können das Bodenfahrzeug, Schienenfahrzeug, Seilbahnfahrzeug, Wasserfahrzeug und/oder das Luftfahrzeug besonders klein sein. Zudem können die Fahrzeuge vorteilhafter Weise zentral gesteuert werden. Dabei kann auch eine automatisierte Steuerung vorgesehen sein. Somit kann vorgesehen sein, dass die Sensoren automatisch dem Bewegungsplan folgen.

Nach einer bevorzugten Ausgestaltung kann vorgesehen sein, dass das Luftfahrzeug von einem Bodenfahrzeug, Schienenfahrzeug, Seilbahnfahrzeug und/oder Wasserfahrzeug transportierbar ist. Das Bodenfahrzeug oder Wasserfahrzeug kann dabei ebenfalls ein mobiler Sensor sein. Somit kann das Luftfahrzeug von dem Bodenfahrzeug oder Wasserfahrzeug aus gestartet werden. Dadurch können von Fahrzeugen schwer oder nicht erreichbare oder versperrte Bereiche erkundet werden und Messungen durchgeführt werden. Dadurch kann die Komplexität der Steuerung der mobilen Sensoren vereinfacht werden.

Es kann vorgesehen sein, dass der mobile Sensor Navigationssensoren aufweisen. Insbesondere kann vorgesehen sein, dass der mobile Sensor zumindest einen von GPS, Radarsystem, LIDAR, Laser und optische Sensoren aufweist. Somit kann der Sensor für die Navigation benötigte Daten selbst bereitstellen. Es kann vorgesehen sein, dass dem Sensor Navigationsdaten bereitgestellt werden. Der Sensor kann die bereitgestellten Navigationsdaten und die Navigationssensoren zur Navigation verwenden.

Es kann vorgesehen sein, dass der mobile Sensor explosionsgeschützt ist. Somit kann der mobile Sensor auch in explosionsgefährdeten Bereichen eingesetzt werden.

Mit der Erfindung wird ferner die Verwendung des Verfahrens zur Qualitätsüberwachung und Feststellung einer Kontamination eines Raums vorgeschlagen, wobei der Raum ein durch Raumkoordinaten bestimmbares Volumen ist, welcher ein Fluid aufweist oder der im Wesentlichen mit einem Fluid gefüllt ist. Es kann vorgesehen sein, das Verfahren zu verwenden für die Überwachung von:
- Chemie- und Industriegelände und Anlagen/Betriebe/Einrichtungen (z.B. Raffinerien, Lager, Kläranlagen, Pipelines (oberirdisch, erdverlegt, in Gewässern verlegt)
- Verkehrsinfrastruktur (z.B. Straßen, Häfen, Flughäfen, Bahnhöfe, Gleisanlagen,...)
- Städtische Bereiche, öffentliche Einrichtungen (Behörden, Kindergärten, Schulen, Universitäten, Forschungszentren, Krankenhäuser...)
- Kritische Infrastruktur, Versorgungseinrichtungen (z.B. Pipeline, Kanäle, Wasserstraßen)
- Militärische Einrichtungen, Strahlenschutzbereiche
- Kulturgüter, Naturschutzgebiete, Landschaftsschutzgebiete, Weltkulturerbe, Tierschutzgebiete, beispielsweise können Sensorsysteme auch von Tieren getragen werden, natürliche Emissionsquellen wie z.B. unterirdische Kohlebrände, Ölteppiche, Vulkane etc.
- Siedlungsgebiete von Menschen und Tieren, Küstengebiete, Veranstaltungen (Sport, Kultur, etc.)
- Mastbetriebsgelände, Tierhaltungsbetriebe, Weideplätze,
- Gewässern (Ölteppiche, Mikroplastik)
- Silvesteraktivitäten, Feuerspiele, Feuerdarbietungen, Vulkanaktivitäten, Waldbrände, Wüstenstürme, Stürme mit Erosionserscheinungen, Schneestürme, Pollenflug (Allergiker), Staubbelastungen, Sprühnebel von z.B. Pflanzenschutzmitteln.

## Patentansprüche

1. Verfahren zur Qualitätsüberwachung und Feststellung einer Kontamination eines Raums, wobei der Raum ein durch Raumkoordinaten bestimmbares Volumen ist, welcher ein Fluid aufweist oder der im Wesentlichen mit einem Fluid gefüllt ist, aufweisend die Verfahrensschritte,
a) Messen einer Mehrzahl von Qualitätsparameter-Istwerten in einem Raum mit einem Sensorsystem, wobei der Raum ein durch Raumkoordinaten bestimmbares Volumen ist, welcher ein Fluid aufweist oder der im Wesentlichen mit einem Fluid gefüllt ist, wobei der Istwert von zumindest einem Qualitätsparameter an einer Mehrzahl von Messorten gemessen wird, wobei das Sensorsystem eine Mehrzahl von Sensoren umfasst, wobei die Sensoren mobile Sensoren sind, oder zumindest ein Sensor ein stationärer Sensor und zumindest ein Sensor ein mobiler Sensor ist, wobei jeder Sensor an einer mittels Raumkoordinaten bestimmten Position des Messortes den zumindest einen Qualitätsparameter-Istwert misst,
b) Übertragen der an einer Mehrzahl von Messorten gemessenen Qualitätsparameter-Istwerte an ein Datenverarbeitungssystem,
c) Abgleich der an den jeweiligen Messorten gemessenen Qualitätsparameter-Istwerte mit in dem Datenverarbeitungssystem für die jeweiligen Messorte hinterlegten Sollwerten,
d) Bewegen des zumindest einen mobilen Sensors mit einem Steuersystem an einen von dem vorherigen Messort des zumindest einen mobilen Sensors beabstandeten Messort,
wobei der zumindest eine mobile Sensor verschiedene Messorte nach einem in dem Datenverarbeitungssystem hinterlegten Bewegungsplan ansteuert,
**dadurch gekennzeichnet, dass**
bei einem Störungsfall, bei dem wenigstens ein Istwert von dem für den jeweiligen Messort definierten Sollwert abweicht, zumindest ein mobiler Sensor mit dem Steuersystem von dem hinterlegten Bewegungsplan abweichend bewegt wird und in dem Raum um die Position des Messortes mit der detektierten Istwertabweichung herum, zusätzliche Qualitätsparameter-Istwerte an zusätzlichen Messorten misst und an das Datenverarbeitungssystem überträgt, wobei das Datenverarbeitungssystem die räumliche Ausbreitung, Art der Kontamination und/oder Konzentration der jeweiligen Kontamination des Raums bestimmt.

2. Verfahren nach dem vorherigen Anspruch, wobei in Schritt a) zusätzlich weitere Parameter, insbesondere Windrichtung, Windstärke, Lufttemperatur, Luftfeuchtigkeit und/oder Luftdruck gemessen werden, und in Schritt b) an das Datenverarbeitungssystem übertragen werden.

3. Verfahren nach einem der vorherigen Ansprüche, wobei der zumindest eine Qualitätsparameter-Istwert die Konzentration einer Substanz in einem Fluid, insbesondere der Luft ist, insbesondere die Konzentration eines Gefahrstoffes und/oder Schadstoffes.

4. Verfahren nach einem der vorherigen Ansprüche, wobei dem Datenverarbeitungssystem weitere Umgebungsdaten bereitgestellt werden, insbesondere Informationen über mögliche Emissionsquellen.

5. Verfahren nach einem der vorherigen Ansprüche, wobei das Messen eines Qualitätsparameter-Istwertes eine Probennahme umfasst.

6. Verfahren nach einem der vorherigen Ansprüche, wobei der Bewegungsplan ortsfeste Objekte mit ihren Raumkoordinaten aufweist zu denen ein Minimalabstand und/oder Maximalabstand für die Annäherung des mobilen Sensors hinterlegt ist.

7. Verfahren nach einem der vorherigen Ansprüche, wobei
a) im Bewegungsplan Messorte definiert sind, die wenigstens ein mobiler Sensor in einer vorgegebenen Reihenfolge oder statistisch ansteuert; und/oder
b) wenigstens ein mobiler Sensor/en die Messorte innerhalb des zu überwachenden Raums zufällig auswählt.

8. Verfahren nach einem der vorherigen Ansprüche, wobei wenigstens ein mobiler Sensor im Störungsfall den Ort der höchsten Konzentration der über dem Sollwert liegenden Substanz im Raum ermittelt.

9. Verfahren nach einem der vorherigen Ansprüche, wobei das Datenverarbeitungssystem bei einer Überschreitung des Ist-Wertes vom Soll-Wert und abhängig von der Ausbreitung und Art der Überschreitung des Ist-Wertes vom Soll-Wert automatische Warnungen ausgibt, wobei das Datenverarbeitungssystem wieder Entwarnung gibt, wenn die Qualitätsparameter-Istwert/e den im Datenverarbeitungssystem, für den jeweils mittels stationärem Sensor/en und/oder mobilem Sensor/en untersuchten Raum, hinterlegten Sollwert/en, entsprechen.

10. Verfahren nach einem der vorherigen Ansprüche, wobei das Datenverarbeitungssystem bei einer Überschreitung des Ist-Wertes vom Soll-Wert entsprechend eines diesbezüglich im Datenverarbeitungssystem hinterlegten Maßnahmenplans Gefahrenabwehrmaßnahmen vorschlägt und/oder durchführt, insbesondere ein Abschalten möglicher Emissionsquellen und/oder eine Absperrung von Gefahrenbereichen.

11. Verfahren nach einem der vorherigen Ansprüche, wobei der mobile Sensor abhängig vom jeweiligen Messort und auf Basis des gemessenen Qualitätsparameter-Istwert, diese Daten an das Datenverarbeitungssystem zur Erstellung eines Sollwert-Profils für unterschiedliche Messorte des Raums sendet.

12. System zur Qualitätsüberwachung und Feststellung einer Kontamination eines Raums, wobei der Raum ein durch Raumkoordinaten bestimmbares Volumen ist, das im Wesentlichen mit einem Fluid gefüllt ist, aufweisend ein Sensorsystem, ein Datenverarbeitungssystem und ein Steuersystem,
wobei das Sensorsystem eine Mehrzahl von Sensoren aufweist, wobei die Sensoren mobile Sensoren sind, oder zumindest ein Sensor ein stationärer Sensor und zumindest ein Sensor ein mobiler Sensor ist, wobei das Sensorsystem dazu eingerichtet ist eine Mehrzahl von Qualitätsparameter-Istwerten in einem Raum zu messen, wobei der Raum ein durch Raumkoordinaten bestimmbares Volumen ist, der ein Fluid aufweist oder der im Wesentlichen mit einem Fluid gefüllt ist, wobei der Istwert von zumindest einem Qualitätsparameter an einer Mehrzahl von Messorten gemessen wird, wobei jeder Sensor an einer mittels Raumkoordinaten bestimmten Position des Messortes den zumindest einen Qualitätsparameter-Istwert misst,
wobei das Sensorsystem dazu eingerichtet ist, die an einer Mehrzahl von Messorten gemessenen Qualitätsparameter-Istwerte an das Datenverarbeitungssystem zu übertragen,
wobei das Datenverarbeitungssystem dazu eingerichtet ist, die an einer Mehrzahl von Messorten gemessenen Qualitätsparameter-Istwerte mit für den jeweiligen Messort bereitgestellten Sollwerten zu vergleichen,
wobei das Steuersystem dazu eingerichtet ist, den zumindest einen mobilen Sensor an einen von dem vorherigen Messort des zumindest einen mobilen Sensors beabstandeten Messort zu bewegen, wobei der zumindest eine mobile Sensor verschiedene Messorte nach einem in dem Datenverarbeitungssystem hinterlegten Bewegungsplan ansteuert,
**dadurch gekennzeichnet, dass**
bei einem Störungsfall, bei dem wenigstens ein Istwert von dem für den jeweiligen Messort definierten Sollwert abweicht, zumindest ein mobiler Sensor mit dem Steuersystem von dem hinterlegten Bewegungsplan abweichend bewegt wird und in dem Raum um die Position des Messortes mit der detektierten Istwertabweichung herum, zusätzliche Qualitätsparameter-Istwerte an zusätzlichen Messorten misst und an das Datenverarbeitungssystem überträgt, wobei das Datenverarbeitungssystem die räumliche Ausbreitung, Art der Kontamination und/oder Konzentration der jeweiligen Kontamination des Raums bestimmt.

13. System nach dem vorherigen Anspruch, wobei der mobil angeordnete Sensor ein Bodenfahrzeug, Schienenfahrzeug, Seilbahnfahrzeug, Wasserfahrzeug und/oder ein Luftfahrzeug ist, wobei das System insbesondere zumindest zwei mobil angeordnete Sensoren aufweist und ein mobil angeordneter Sensor ein Bodenfahrzeug, Schienenfahrzeug, Seilbahnfahrzeug oder ein Wasserfahrzeug und ein anderer mobil angeordneter Sensor ein Luftfahrzeug, insbesondere eine Drohne, ist.

14. System nach dem vorherigen Anspruch, wobei das Bodenfahrzeug, Schienenfahrzeug, Seilbahnfahrzeug, Wasserfahrzeug und/oder das Luftfahrzeug autonom betreibbar ist, insbesondere in den Autonomiestufen 4 oder 5.

15. Verwendung des Verfahrens nach einem der Ansprüche 1 bis 11 zur Qualitätsüberwachung und Feststellung einer Kontamination eines Raums, wobei der Raum ein durch Raumkoordinaten bestimmbares Volumen ist, der ein Fluid aufweist oder der im Wesentlichen mit einem Fluid gefüllt ist.

## Claims

1. Method for quality monitoring and detecting the contamination of a space, wherein the space is a volume which can be determined by spatial coordinates and which comprises a fluid or which is substantially filled with a fluid, comprising the method steps:
a) measuring a plurality of quality parameter actual values in a space by use of a sensor system, wherein the space is a volume which can be determined by spatial coordinates and which comprises a fluid or which is substantially filled with a fluid, wherein the actual value of at least one quality parameter is measured at a plurality of measurement locations, wherein the sensor system comprises a plurality of sensors, wherein the sensors are mobile sensors, or at least one sensor is a stationary sensor and at least one sensor is a mobile sensor, wherein each sensor measures the at least one actual quality parameter value at a position of the measurement location determined by means of spatial coordinates;
b) transmitting the quality parameter actual values measured at a plurality of measurement locations to a data processing system;
c) matching the quality parameter actual values measured at the respective measurement locations with target values stored in the data processing system for the respective measurement locations,
d) moving the at least one mobile sensor by means of a control system to a measurement location spaced apart from the previous measurement location of the at least one mobile sensor, wherein the at least one mobile sensor approaches different measurement locations according to a movement plan stored in the data processing system,
**characterized in that**
in the event of a malfunction in which at least one actual value deviates from the target value defined for the respective measurement location, at least one mobile sensor is moved by the control system in a manner deviating from the stored movement plan and measures in the space around the position of the measurement location with the detected actual value deviation additional quality parameter actual values at additional measurement locations and transmits them to the data processing system, wherein the data processing system determines the spatial spread, type of contamination and/or concentration of the respective contamination of the space.

2. Method according to the preceding claim, wherein in step a) further parameters, in particular wind direction, wind strength, air temperature, air humidity and/or air pressure, are measured and transmitted in step b) to the data processing system.

3. Method according to any one of the preceding claims, wherein the at least one quality parameter actual value is the concentration of a substance in a fluid, in particular the air, in particular the concentration of a hazardous substance and/or pollutant.

4. Method according to any one of the preceding claims, wherein further environmental data are provided to the data processing system, in particular information about possible emission sources.

5. Method according to any one of the preceding claims, wherein the measuring of a quality parameter actual value comprises a sampling.

6. Method according to any one of the preceding claims, wherein the movement plan includes stationary objects with their spatial coordinates to which a minimum distance and/or a maximum distance for the approach of the mobile sensor are stored.

7. Method according to any one of the preceding claims, wherein
a) measurement locations are defined in the movement plan, which are approached by at least one mobile sensor in a predetermined sequence or statistically; and/or
b) at least one mobile sensor randomly select(s) the measurement locations within the space to be monitored.

8. Method according to any one of the preceding claims, wherein at least one mobile sensor determines the location of the highest concentration of the substance above the target value in the space in the event of a malfunction.

9. Method according to any one of the preceding claims, wherein the data processing system outputs automatic warnings if the actual value exceeds the target value and depending on the spread and type of exceedance of the actual value from the target value, wherein the data processing system gives the all-clear signal again if the quality parameter actual value(s) respectively correspond(s) to the target value(s) stored in the data processing system for the respective space examined by means of stationary sensor(s) and/or mobile sensor(s).

10. Method according to any one of the preceding claims, wherein the data processing system, in the event that the actual value exceeds the target value, proposes and/or carries out danger prevention measures, in particular a shutdown of possible emission sources and/or a cordoning off of danger areas according to a corresponding action plan stored in the data processing system.

11. Method according to any one of the preceding claims, wherein the mobile sensor, depending on the respective measurement location and on the basis of the measured quality parameter actual value, transmits these data to the data processing system for creating a target value profile for different measurement locations of the space.

12. System for quality monitoring and detecting contamination of a space, wherein the space is a volume determinable by space coordinates and is substantially filled with a fluid, comprising a sensor system, a data processing system, and a control system,
wherein the sensor system comprises a plurality of sensors, wherein the sensors are mobile sensors, or wherein at least one sensor is a stationary sensor and at least one sensor is a mobile sensor, wherein the sensor system is adapted to measure a plurality of quality parameter actual values in a space, wherein the space is a volume determinable by spatial coordinates and comprises a fluid or is substantially filled with a fluid, wherein the actual value of at least one quality parameter is measured at a plurality of measurement locations, wherein each sensor measures the at least one quality parameter at a position of the measurement location determined by means of spatial coordinates,
wherein the sensor system is adapted to transmit the quality parameter actual values measured at a plurality of measurement locations to the data processing system,
wherein the data processing system is adapted to compare the quality parameter actual values measured at a plurality of measurement locations with target values provided for the respective measurement location,
wherein the control system is adapted to move the at least one mobile sensor to a measurement location spaced from the previous measurement location of the at least one mobile sensor, wherein the at least one mobile sensor approaches different measurement locations according to a movement plan stored in the data processing system,
**characterized in that**,
in the event of a malfunction in which at least one actual value deviates from the target value defined for the respective measurement location, at least one mobile sensor is moved by the control system in a manner deviating from the stored movement plan and measures additional quality parameter actual values at additional measurement locations in the space around the position of the measurement location exhibiting the detected actual value deviation and transmits them to the data processing system, wherein the data processing system determines the spatial spread, type of contamination and/or concentration of the respective contamination of the space.

13. System according to the preceding claim, wherein the mobile sensor is a ground vehicle, a rail vehicle, a cable car vehicle, a watercraft and/or an aircraft, wherein the system in particular comprises at least two mobile sensors and one mobile sensor is a ground vehicle, a rail vehicle, a cable car vehicle or a watercraft and another mobile sensor is an aircraft, in particular a drone.

14. System according to the preceding claim, wherein the ground vehicle, the rail vehicle, the cable car vehicle, the watercraft and/or the aircraft can be operated autonomously, in particular in the autonomy levels 4 or 5.

15. Use of the method according to any one of claims 1 to 11 for quality monitoring and detection of contamination of a space, wherein the space is a volume determinable by spatial coordinates and comprises a fluid or is substantially filled with a fluid.

## Revendications

1. Procédé pour le contrôle de qualité et la détermination d'une contamination d'un espace, dans lequel l'espace est un volume déterminable par des coordonnées spatiales, l'espace contenant un fluide ou étant sensiblement rempli par un fluide, le procédé comprenant les étapes de :
a) mesure d'une pluralité de valeurs réelles de paramètres de qualité dans un espace au moyen d'un système de capteurs, dans lequel l'espace est un volume déterminable par des coordonnées spatiales, l'espace contient un fluide ou est sensiblement rempli par un fluide, la valeur réelle d'au moins un paramètre de qualité est mesurée dans une pluralité d'emplacements de mesure, le système de capteurs comprend une pluralité de capteurs, les capteurs sont des capteurs mobiles, ou au moins un capteur est un capteur stationnaire et au moins un capteur est un capteur mobile, chaque capteur mesure la valeur réelle de l'au moins un paramètre de qualité à une position de l'emplacement de mesure déterminée par des coordonnées spatiales,
b) transmission des valeurs réelles de paramètres de qualité mesurées à une pluralité d'emplacements de mesure à un système de traitement de données,
c) comparaison des valeurs réelles de paramètres de qualité mesurées aux emplacements de mesure respectifs avec des valeurs cibles stockées dans le système de traitement de données pour les emplacements de mesure respectifs,
d) déplacement de l'au moins un capteur mobile avec un système de commande vers un autre emplacement de mesure espacé de l'emplacement de mesure respectif de l'au moins un capteur mobile,
dans lequel l'au moins un capteur mobile se dirige vers différents emplacements de mesure selon un plan de déplacement stocké dans le système de traitement de données,
**caractérisé en ce que**
en cas d'incident dans lequel au moins une valeur réelle s'écarte de la valeur cible définie pour l'emplacement de mesure respectif, au moins un capteur mobile est déplacé avec le système de commande s'écartant du plan de déplacement stocké et, dans l'espace autour de la position de l'emplacement de mesure avec l'écart de valeur réelle détecté, mesure des valeurs réelles de paramètres de qualité supplémentaires à des emplacements de mesure supplémentaires et les transmet au système de traitement de données, le système de traitement de données déterminant la répartition spatiale, le type de contamination et/ou la concentration de la pollution respective de l'espace.

2. Procédé selon la revendication précédente, dans lequel à l'étape a) d'autres paramètres supplémentaires, en particulier la direction du vent, la force du vent, la température de l'air, l'humidité de l'air et/ou la pression de l'air sont mesurés, et à l'étape b) ils sont transmis au système de traitement de données.

3. Procédé selon l'une quelconque des revendications précédentes, dans lequel la valeur réelle de l'au moins un paramètre de qualité est la concentration d'une substance dans le fluide, en particulier l'air, notamment la concentration d'une substance dangereuse et/ou d'un polluant.

4. Procédé selon l'une quelconque des revendications précédentes, dans lequel d'autres données environnementales sont fournies au système de traitement de données, en particulier des informations sur d'éventuelles sources d'émission.

5. Procédé selon l'une quelconque des revendications précédentes, dans lequel la mesure d'une valeur réelle de paramètres de qualité comprend un échantillonnage.

6. Procédé selon l'une quelconque des revendications précédentes, dans lequel le plan de déplacement comprend des objets stationnaires avec leurs coordonnées spatiales auxquelles une distance minimale et/ou une distance maximale pour l'approche du capteur mobile est stockée.

7. Procédé selon l'une quelconque des revendications précédentes, dans lequel
a) des emplacements de mesure sont définis dans le plan de déplacement vers lesquels au moins un capteur mobile se dirige dans une séquence prédéterminée ou statistiquement ; et/ou
b) au moins un capteur mobile sélectionne aléatoirement les emplacements de mesure à l'intérieur de l'espace à contrôler.

8. Procédé selon l'une quelconque des revendications précédentes, dans lequel au moins un capteur mobile détermine l'emplacement de la concentration la plus élevée de la substance au-dessus de la valeur cible dans l'espace en cas d'incident.

9. Procédé selon l'une quelconque des revendications précédentes, dans lequel le système de traitement de données émet des avertissements automatiques lorsque la valeur réelle dépasse la valeur cible et en fonction de la propagation et du type de dépassement de la valeur réelle par rapport à la valeur cible, dans lequel le système de traitement de données donne un signal de fin d'alerte lorsque la valeur réelle ou les valeurs réelles de paramètres de qualité correspondent) à la valeur cible ou aux valeurs cibles stockées dans le système de traitement de données pour l'espace examiné par le ou les capteurs stationnaires et/ou mobiles.

10. Procédé selon l'une quelconque des revendications précédentes, dans lequel le système de traitement de données, si la valeur réelle dépasse la valeur cible, propose et/ou exécute des mesures de prévention de risques conformément à un plan d'action stocké dans le système de traitement de données, en particulier un arrêt d'éventuelles sources d'émission et/ou un barrage de zones dangereuses.

11. Procédé selon l'une quelconque des revendications précédentes, dans lequel le capteur mobile, en fonction de l'emplacement de mesure respectif et sur la base de la valeur réelle du paramètre de qualité mesurée, envoie ces données au système de traitement de données pour créer un profil de valeur cible pour différents emplacements de mesure de l'espace.

12. Système pour le contrôle de qualité et la détermination d'une contamination d'un espace, dans lequel l'espace est un volume déterminable par des coordonnées spatiales, qui est sensiblement rempli d'un fluide, le système comprenant : un système de capteur, un système de traitement de données et un système de commande,
dans lequel le système de capteur comprend une pluralité de capteurs, les capteurs sont des capteurs mobiles, ou au moins un capteur est un capteur stationnaire et au moins un capteur est un capteur mobile, le système de capteur est configuré pour mesurer une pluralité de valeurs réelles de paramètres de qualité dans l'espace, l'espace est un volume déterminable par des coordonnées spatiales, qui comprend un fluide ou est sensiblement rempli d'un fluide, la valeur réelle d'au moins un paramètre de qualité est mesurée à une pluralité d'emplacements de mesure, chaque capteur mesure la valeur réelle de l'au moins un paramètre de qualité à une position de l'emplacement de mesure déterminée par des coordonnées spatiales,
dans lequel le système de capteur est configuré pour transmettre les valeurs réelles de paramètres de qualité mesurées à une pluralité d'emplacements de mesure au système de traitement de données,
dans lequel le système de traitement de données est configuré pour comparer les valeurs réelles de paramètres de qualité mesurées à une pluralité d'emplacements de mesure avec des valeurs cibles fournies pour l'emplacement de mesure respectif,
dans lequel le système de commande est configuré pour déplacer l'au moins un capteur mobile vers un autre emplacement de mesure espacé de l'emplacement de mesure respectif de l'au moins un capteur mobile,
dans lequel l'au moins un capteur mobile se dirige vers différents emplacements de mesure selon un plan de déplacement stocké dans le système de traitement de données,
**caractérisé en ce que**
en cas d'incident dans lequel au moins une valeur réelle s'écarte de la valeur cible définie pour l'emplacement de mesure respectif, au moins un capteur mobile est déplacé avec le système de commande s'écartant du plan de déplacement stocké et, dans l'espace autour de la position de l'emplacement de mesure avec l'écart de valeur réelle détecté, mesure des valeurs réelles de paramètres de qualité supplémentaires à des emplacements de mesure supplémentaires et les transmet au système de traitement de données, le système de traitement de données déterminant la répartition spatiale, le type de contamination et/ou la concentration de la pollution respective de l'espace.

13. Système selon la revendication précédente, dans lequel le capteur agencé mobile est un véhicule terrestre, un véhicule ferroviaire, un véhicule téléphérique, un véhicule aquatique et/ou un véhicule aérien, dans lequel le système comprend en particulier au moins deux capteurs agencés mobiles et un capteur agencé mobile est un véhicule terrestre, un véhicule ferroviaire, un véhicule téléphérique ou un véhicule aquatique et un autre capteur agencé mobile est un véhicule aérien, en particulier un drone.

14. Système selon la revendication précédente, dans lequel le véhicule terrestre, le véhicule ferroviaire, le véhicule téléphérique, le véhicule aquatique et/ou le véhicule aérien peut être conduit de façon autonome, en particulier est de niveau d'autonomie 4 ou 5.

15. Utilisation du procédé selon l'une quelconque des revendications 1 à 11 pour le contrôle de qualité et la détermination d'une contamination d'un espace, dans laquelle l'espace est un volume déterminable par des coordonnées spatiales, l'espace contenant un fluide ou étant sensiblement rempli par un fluide.
